# EUROPEAN PATENT APPLICATION

(11) **EP 3 805 392 A2**
(43) Date of publication of application: **14.04.2021**
(21) Application number: 20829298.7
(22) Date of filing: 19.08.2020
(51) Int. Cl.: C12N 15/50, C12N 15/85, C12N 15/861, A61K 39/215, A61P 31/14, A61P 31/20

(54) **NUCLEIC ACID SEQUENCE EXPRESSING SARS-COV-2 VIRUS ANTIGEN PEPTIDE AND USE THEREOF**

(30) Priority: 23.02.2020 CN 202010110070; 05.03.2020 CN 202010145657
(71) Applicant: Guangzhou N Biomed Ltd., Guangzhou, Guangdong 510663 (CN)
(72) Inventor: CHEN, Ling, Guangzhou, Guangdong 510663 (CN); GUAN, Suhua, Guangzhou, Guangdong 510663 (CN); YANG, Chenchen, Guangzhou, Guangdong 510663 (CN); LIU, Xiaolin, Guangzhou, Guangdong 510663 (CN)
(74) Representative: Gassner, Birgitta
(86) International application number: PCT/CN2020/110086
(87) International publication number: WO 2021/000969

(57) **Abstract**

The present disclosure discloses nucleotide sequences as shown by SEQ ID NO: 1, SEQ ID NO: 2 and the nucleotide sequences having at least 90% homology therewith, which encode peptide antigens of SARS-CoV-2 virus. According to some embodiments of the present disclosure, the nucleotide sequences can be effectively expressed in human cells and produce the corresponding peptides, thereby inducing an immune-protective response accordingly. Thus, the nucleotide sequences of the present disclosure are expected to be developed as a vaccine against SARS-CoV-2 infection.

SEQUENCE LISTING

<110>  GUANGZHOU N BIOMED LTD.

<120>  NUCLEOTIDE SEQUENCES ENCODING PEPTIDE ANTIGENS OF SARS-COV-2
       VIRUS AND USE THEREOF

<130>  P20GZ1NW00079US

<150>  CN202010110070.8
<151>  2020-02-23

<150>  CN202010145657.2
<151>  2020-03-05

<160>  2

<170>  PatentIn version 3.5

<210>  1
<211>  3822
<212>  DNA
<213>  Artificial Sequence

<220>
<223>  Optimized nucleotide sequence

<400>  1
atgttcgtgt ttctggtgct gctgcctctg gtgagctccc agtgcgtgaa cctgaccaca      60

aggacccagc tgccacctgc ctataccaat agcttcacac ggggcgtgta ctatcccgac     120

aaggtgttta gatctagcgt gctgcactcc acccaggatc tgtttctgcc tttcttttct     180

aacgtgacat ggttccacgc catccacgtg tccggcacca atggcacaaa gcggttcgac     240

aatccagtgc tgccctttaa cgatggcgtg tacttcgcct ccaccgagaa gtctaacatc     300

atcagaggct ggatctttgg caccacactg gacagcaaga cccagtccct gctgatcgtg     360

aacaatgcca caaacgtggt catcaaggtg tgcgagttcc agttttgtaa tgatcccttc     420

ctgggcgtgt actatcacaa gaacaataag tcttggatgg agagcgagtt tagggtgtat     480

tcctctgcca acaattgcac ctttgagtac gtgagccagc ctttcctgat ggacctggag     540

ggcaagcagg gcaatttcaa gaacctgagg gagttcgtgt ttaagaatat cgatggctac     600

ttcaagatct actccaagca cacaccaatc aacctggtgc gcgacctgcc acagggcttc     660

tctgccctgg agccactggt ggatctgccc atcggcatca acatcacccg gtttcagaca     720

ctgctggccc tgcacagaag ctacctgacc ccaggcgaca gctcctctgg atggacagca     780

ggagctgccg cctactatgt gggctatctg cagccccgca ccttcctgct gaagtacaac     840

gagaatggca ccatcacaga cgcagtggat tgcgccctgg accccctgtc tgagaccaag     900

tgtacactga agagctttac agtggagaag ggcatctacc agaccagcaa cttcagggtg     960

cagccaacag agtccatcgt gcgctttccc aatatcacca acctgtgccc ttttggcgag    1020

gtgttcaatg ccacacgctt cgccagcgtg tacgcctgga ataggaagcg catctccaac    1080

tgcgtggccg actattctgt gctgtacaac agcgcctcct tctctacctt taagtgttat 1140

ggcgtgagcc ccaccaagct gaatgatctg tgctttacaa acgtgtacgc cgattccttc 1200

gtgatcaggg gcgacgaggt gcgccagatc gcaccaggac agaccggcaa gatcgcagac 1260

tacaattata agctgcctga cgatttcaca ggctgcgtga tcgcctggaa ctctaacaat 1320

ctggatagca aagtgggcgg caactacaat tatctgtacc ggctgtttag aaagtctaat 1380

ctgaagccat tcgagcggga catctccacc gagatctacc aggccggctc tacaccctgc 1440

aatggcgtgg agggctttaa ctgttatttc cctctgcagt cctacggctt ccagccaacc 1500

aacggcgtgg gctatcagcc ctacagagtg gtggtgctgt cttttgagct gctgcacgca 1560

cctgcaaccg tgtgcggccc aaagaagagc acaaatctgg tgaagaacaa gtgcgtgaac 1620

ttcaacttca acggactgac cggcacaggc gtgctgaccg agagcaacaa gaagttcctg 1680

ccatttcagc agttcggcag ggacatcgca gataccacag acgccgtgcg cgaccctcag 1740

accctggaga tcctggacat cacaccatgt tccttcggcg gcgtgtctgt gatcacccca 1800

ggcaccaata catccaacca ggtggccgtg ctgtatcagg acgtgaattg cacagaggtg 1860

cccgtggcaa tccacgcaga tcagctgacc cctacatggc gggtgtactc taccggcagc 1920

aacgtgttcc agacaagagc cggatgcctg atcggagcag agcacgtgaa caatagctat 1980

gagtgcgaca tccctatcgg cgccggcatc tgtgcctcct accagaccca gacaaactcc 2040

ccaaggagag cccggtctgt ggccagccag tccatcatcg cctataccat gagcctgggc 2100

gccgagaaca gcgtggccta ctccaacaat tctatcgcca tccctaccaa cttcacaatc 2160

agcgtgacca cagagatcct gccagtgagc atgaccaaga catccgtgga ctgcaccatg 2220

tatatctgtg gcgattccac agagtgttct aacctgctgc tgcagtacgg ctccttttgc 2280

acccagctga atagagccct gacaggcatc gccgtggagc aggacaagaa cacccaggag 2340

gtgttcgccc aggtgaagca gatctacaag acaccaccca tcaaggactt tggcggcttc 2400

aacttcagcc agatcctgcc cgatcctagc aagccatcca agcggtcttt tatcgaggac 2460

ctgctgttca acaaggtgac cctggccgat gccggcttca tcaagcagta tggcgattgt 2520

ctgggcgaca tcgccgccag agacctgatc tgcgcccaga agtttaatgg cctgaccgtg 2580

ctgcctccac tgctgacaga tgagatgatc gcacagtaca cctctgccct gctggccggc 2640

accatcacaa gcggatggac attcggcgca ggagccgccc tgcagatccc ctttgccatg 2700

cagatggcct atcggttcaa cggcatcggc gtgacccaga atgtgctgta cgagaaccag 2760

aagctgatcg ccaatcagtt taacagcgcc atcggcaaga tccaggactc tctgagctcc 2820

accgccagcg ccctgggcaa gctgcaggat gtggtgaatc agaacgccca ggccctgaat 2880

acactggtga agcagctgtc tagcaacttc ggcgccatct cctctgtgct gaatgacatc 2940

ctgagccggc tggacaaggt ggaggcagag gtgcagatcg accggctgat caccggcaga 3000

ctgcagtccc tgcagaccta cgtgacacag cagctgatca gggcagcaga gatcagggcc 3060

tctgccaatc tggccgccac aaagatgagc gagtgcgtgc tgggacagtc caagagggtg 3120

gacttttgcg gcaagggcta tcacctgatg agcttcccac agtccgcccc tcacggagtg 3180

gtgtttctgc acgtgaccta cgtgccagcc caggagaaga acttcaccac agcccccgcc 3240

atctgtcacg atggcaaggc ccactttcct agggagggcg tgttcgtgag caacggcacc 3300

cactggtttg tgacacagcg caatttctac gagccacaga tcatcaccac agacaatacc 3360

ttcgtgtccg gcaactgcga cgtggtcatc ggcatcgtga acaatacagt gtatgatcct 3420

ctgcagccag agctggactc ttttaaggag gagctggata agtacttcaa gaatcacacc 3480

agccccgacg tggatctggg cgacatctct ggcatcaatg ccagcgtggt gaacatccag 3540

aaggagatcg acagactgaa cgaggtggcc aagaatctga acgagagcct gatcgatctg 3600

caggagctgg gcaagtatga gcagtacatc aagtggccct ggtatatctg gctgggcttc 3660

atcgccggcc tgatcgccat cgtgatggtg accatcatgc tgtgctgtat gacaagctgc 3720

tgttcctgcc tgaagggctg ctgttcttgt ggcagctgct gtaagtttga tgaggacgat 3780

tccgagcctg tgctgaaggg cgtgaagctg cactacacct aa 3822

<210> 2
<211> 3825
<212> DNA
<213> Artificial Sequence

<220>
<223> Optimized nucleotide sequence

<400> 2
atgtttgtct tcctggtgct gctgcctctg gtctcttctc agtgtgtgaa cctgaccacc 60

aggacacagc tgcctcctgc ttacaccaac tccttcacac ggggcgtcta ctaccctgac 120

aaggtcttca ggtcctctgt gctgcactcc acacaagacc tgttcctgcc attcttctcc 180

aacgtgacct ggttccatgc catccatgtc tctggcacca atggcaccaa gaggttcgac 240

aaccctgtgc tgccattcaa cgacggcgtc tactttgcct ccaccgagaa gtccaacatc 300

atcaggggct ggatctttgg caccaccctg gactccaaga cccagtccct gctgattgtg 360

aacaatgcca ccaacgtggt gatcaaagtc tgcgagttcc agttctgcaa tgacccattc 420

ctgggcgtct actaccacaa gaacaacaag tcctggatgg agtctgagtt cagggtctac 480

tcctctgcca acaactgcac ctttgaatat gtctcccagc cattcctgat ggacctggag 540

ggcaagcagg gcaacttcaa gaacctgagg gagttcgtct tcaagaacat cgacggctac 600

ttcaagatct actccaagca cacacccatt aatctcgtcc gggatctgcc tcagggcttc 660

tctgccctgg agcccctggt ggacctgccc attggcatca acatcacacg ctttcagacc 720

ctgctggctc tgcaccggtc ttacctgaca cctggcgact cctcctctgg ctggacagct 780

ggcgctgctg cctactatgt cggctacctg cagcctagga ccttcctgct gaagtacaat 840

gagaatggca ccatcacaga tgctgtggac tgtgccctgg accccctgtc tgagaccaag 900

tgcaccctga agtccttcac agtcgagaag ggcatctacc agacctccaa cttccgggtg 960

cagcccacag agtccattgt caggttcccc aacatcacca acctgtgccc atttggcgag 1020

gtcttcaatg ccacacgctt tgcctctgtc tatgcctgga acaggaagag gatctccaac 1080

tgtgtggctg actactctgt gctgtacaac tctgcctcct tctccacatt taagtgctat 1140

ggcgtctccc ccaccaagct gaatgacctg tgcttcacca atgtctatgc tgactccttt 1200

gtgatccggg gcgatgaagt taggcagatt gcccctggcc agacaggcaa gattgctgac 1260

tacaactaca agctgcctga tgacttcacc ggctgtgtga ttgcctggaa ctccaacaac 1320

ctggactcta aagttggcgg caactacaac tacctgtaca ggctgttcag gaagtccaac 1380

ctgaagccat ttgagaggga catctccaca gagatctacc aggctggctc caccccatgc 1440

aatggcgtgg agggcttcaa ctgctacttc cccctgcaat cctatggctt ccaacccacc 1500

aatggcgtgg gctaccagcc atacagggtg gtggtgctgt cctttgagct gctgcatgcc 1560

cctgccaccg tctgtggccc caagaagtcc accaacctcg tgaagaacaa atgtgtgaac 1620

ttcaacttca acggcctgac aggcacaggc gtgctgacag agtccaacaa gaagttcctg 1680

ccattccaac agtttggcag ggacattgct gacaccaccg atgctgtcag ggacccccag 1740

accctggaga tcctggacat caccccatgc tcctttggcg gcgtctctgt gatcacccct 1800

ggcaccaaca cctctaatca agtcgctgtg ctgtaccagg acgtgaactg cacagaggtt 1860

cctgtggcca tccatgctga ccagctgacc cccacctgga gggtctactc caccggctcc 1920

aatgtcttcc aaacacgggc tggctgcctg attggcgctg agcatgtgaa caactcctat 1980

gagtgtgaca tccccattgg cgctggcatc tgtgcctcct accaaaccca gaccaactcc 2040

cctcggaggg ctcgctctgt ggcttctcag tctatcattg cctacaccat gtccctgggc 2100

gctgagaact ctgtggccta ctccaacaac tccattgcca tccccaccaa cttcaccatc 2160

tctgtgacca ccgagatcct gcctgtctcc atgaccaaga cctctgtgga ctgcaccatg 2220

tacatctgtg gcgactccac agagtgctcc aacctgctgc tgcagtatgg ctccttctgc 2280

acccaactga acagggccct gacaggcatt gctgtggagc aggacaagaa cacccaggag 2340

gtctttgccc aagtcaagca gatctacaag acccccccca tcaaggactt tggcggcttc 2400

aacttctccc aaatcctgcc tgacccatcc aagccatcca agaggtcctt cattgaggac 2460

ctgctgttca acaaggttac cctggctgat gctggcttca tcaagcagta tggcgactgc 2520

ctgggcgaca ttgctgccag ggacctgatc tgtgcccaaa agttcaatgg cctgacagtg 2580

ctgccccccc tgctgaccga tgagatgatt gcccagtaca catctgctct gctggctggc 2640

acaatcacct ctggctggac ctttggcgct ggcgctgccc tgcaaatccc atttgccatg 2700

caaatggcct acaggttcaa tggcattggc gtgacccaga acgtgctgta tgagaaccag 2760

aagctgattg ccaaccagtt caactctgcc attggcaaga tccaggactc cctgtcctcc 2820

accgcctctg ccctgggcaa gctgcaggat gtggtgaacc agaatgccca ggccctgaac 2880

accctggtga agcagctgtc ctccaacttt ggcgccatct cctctgtgct gaatgacatc 2940

ctgtcccggc tggacaaggt ggaggctgag gtgcagattg acaggctgat cacaggcagg 3000

ctgcagtccc tgcaaaccta tgtgacccag cagctgatca gggctgctga gatcagggcc 3060

tctgccaacc tggctgccac caagatgtct gaatgtgtgc tgggccagtc caagagggtg 3120

gacttctgtg gcaagggcta ccatctgatg tccttccccc aatctgcccc ccatggcgtg 3180

gtcttcctgc atgtgaccta tgtgcctgct caggagaaga acttcaccac agcccctgcc 3240

atctgccatg atggcaaggc ccacttccct cgggagggcg tctttgtctc caatggcacc 3300

cactggtttg tgacccagag gaacttctat gagcctcaga tcatcaccac agacaacacc 3360

tttgtctctg gcaactgtga tgtggtgatt ggcattgtga acaacacagt ctatgacccc 3420

ctgcagcctg agctggactc cttcaaagag gagctggaca agtacttcaa gaaccacacc 3480

tcccctgatg tggacctggg cgacatctct ggcatcaatg cctctgtggt gaacatccag 3540

aaggagattg acaggctgaa tgaggtggcc aagaacctga atgagtccct gattgacctg 3600

caagagctgg gcaagtatga gcagtacatc aagtggccat ggtacatctg gctgggcttc 3660

attgctggcc tgattgccat tgtgatggtg accatcatgc tgtgctgcat gacctcctgc 3720

tgctcctgcc tgaaaggctg ctgctcctgt ggctcctgct gcaagtttga tgaggatgac 3780

tctgagcctg tgctgaaggg cgtgaagctg cactacacct aataa 3825

## Description

### TECHNICAL FIELD

The present disclosure relates to an optimized coronavirus S protein and the use thereof, particularly to nucleotide sequences encoding peptide antigens of SARS-CoV-2 virus and the use thereof.

### BACKGROUND

Novel Severe Acute Respiratory Syndrome Coronavirus 2 (SARS-CoV-2) has a latent period up to 24 days, and is highly infectious. Unlike the virus that caused Severe Acute Respiratory Syndrome (SARS), some cases infected with SARS-CoV-2 have infectivity during the latent period, while some SARS-CoV-2 carriers do not show any obvious symptoms. This increases the difficulty of epidemic prevention and control. As of 20 July 2020, there have been close to 15 million confirmed cases, including deaths of above 600,000, all over the world. So far there is no specific medicine available for coronavirus disease 2019 (COVID-19). To control the epidemic, most countries mandated quarantine and closed unnecessary facilities, which resulted in huge economic loss. Therefore, it has become the most urgent and major need to rapidly develop preventive vaccines that can improve herd-immunity level and block the transmission of the virus.

Currently, there is no specific antiviral medicine and preventive vaccine against this novel coronavirus, in China and other countries. Preventing and blocking the viral transmission are the keys to control the epidemic. It is known that vaccines have played an irreplaceable role in eliminating various infectious diseases. The published results obtained from the genome alignment of a dozen of SARS-CoV-2 viruses showed that the difference among the viruses is very small, and no mutation has been found. Therefore, the outbreak of new epidemic would be greatly contained, if SARS-CoV-2 vaccines are successfully developed.

SARS-CoV-2 has a complete genome as shown by NC_045512.2, in which the sequence of nucleic acids 21563 to 25384 is a coding sequence for Spike protein (S). The spike (S) protein is a major structural protein of a virion, and plays an important role in mediating the binding of the virion to a host cell receptor and inducing neutralizing antibodies. This makes the S protein an ideal vaccine antigen. The S protein comprises two subunits, S1 and S2. When the virus infects, the S1 subunit binds to the receptor, angiotensin-converting enzyme 2 (ACE2), on host cells. Then the S protein is cleaved by proteases found in the host cells, after which the S1 and S2 subunits are separated to accelerate the fusion of the S2 subunit with cell membrane. Vaccines, which use the S protein as an antigen, comprise nucleic acid-, subunit- and virus-vectored vaccines, and the efficacy of such vaccines are determined by the expression level and protein structure of the S protein.

However, it was shown by experiments that the gene coding for the Spike protein of natural SARS-CoV-2 has low expression level in the cells, such as Human embryonic kidney (HEK) 293 cells, commonly used for expressing subunit-vaccines. If a vaccine adopts natural genetic codons to express the S protein as the antigen, this vaccine would be ineffective or have low titer, thereby being unable to fight the virus infection.

### SUMMARY

The present disclosure is intended to provide a nucleotide sequence encoding a peptide which triggers immunogenicity against SARS-CoV-2, as well as the use thereof.

The technical solutions adopted by the present disclosure are as follows.

In a first aspect of the present disclosure, provided is a nucleotide sequence encoding a peptide which triggers immunogenicity against Novel Severe Acute Respiratory Syndrome Coronavirus 2 (SARS-CoV-2), which comprises at least one selected from:
a) a nucleotide sequence as shown by SEQ ID NO: 1; or
b) a nucleotide sequence as shown by SEQ ID NO: 2; or
c) a nucleotide sequence having at least 90%, preferably 95%, 98% or 99% sequence identity with the nucleotide sequence as shown by SEQ ID NO: 1.

In some embodiments, said peptide is capable of:
inducing an immune response in a human body;
generating a biology reporter;
generating a molecule for detection;
regulating a gene function; or
functioning as a therapeutic molecule.

In a second aspect of the present disclosure, provided is an expression vector which comprises the nucleotide sequence according to the first aspect of the present disclosure.

In some embodiments, the vector may be a viral vector.

In some embodiments, the viral vector may be an adenovirus vector.

In a third aspect of the present disclosure, provided is a cell expressing the peptide based on the nucleotide sequence according to the first aspect of the present disclosure.

In a fourth aspect of the present disclosure, provided is a nucleotide composition which comprises the nucleotide sequence according to the first aspect of the present disclosure, or the expression vector according to the second aspect of the present disclosure.

In some embodiments, the nucleotide composition may further comprise at least one of a pharmaceutically-acceptable adjuvant, carrier, diluent or excipient.

In a fifth aspect of the present disclosure, provided is use of the nucleotide composition according to the fourth aspect of the present disclosure in the preparation of an agent for preventing or treating COVID-19.

In a sixth aspect of the present disclosure, provided is a method for preventing or treating COVID-19, which comprises administering a prophylactically or therapeutically effective amount of the nucleotide composition according to the fourth aspect of the present disclosure to a human.

The beneficial effects of the present disclosure are as follows.

The nucleotide sequences according to some embodiments of the present disclosure can be effectively expressed in mammal cells and produce the corresponding peptides, thereby inducing an immune-protective response accordingly. Such nucleotide sequences are expected to be developed as a vaccine against SARS-CoV-2 infection.

### BRIEF DESCRIPTION OF THE DRAWINGS

FIG. 1 shows the expression of S protein after transfecting with a same dose of pVAX-NB1, pVAX-NB2 and pVAX-NB4.
FIG. 2 shows the antibody levels in serum of the mice immunized with pVAX-NB1- and pVAX-NB2-DNA vaccines.

### DETAILED DESCRIPTION

The Spike protein (S) of SARS-CoV-2 has an amino acid sequence as shown by YP_009724390.1, and the natural nucleotide sequence encoding the S protein of SARS-CoV-2 is designated as NB1.

Precursor mRNA in eukaryotic cells can produce various mRNA splice variants through different splicing manners (by selecting different combination of splice sites), eventually leading to different proteins produced from the same gene sequence. This is very unfavorable for the expression of a protein. The inventor performed codon optimization to the wild-type natural nucleotide sequence and removed potential variable cleavage sites based on self-owned technology, thereby ensuring the uniqueness of expressed protein and reducing the difficulty in subsequent protein purification. The optimized nucleotide sequence is designated as NB2, and is as shown by SEQ ID NO: 1:

ATGTTCGTGTTTCTGGTGCTGCTGCCTCTGGTGAGCTCCCAGTGCGTGAACCTGACC
ACAAGGACCCAGCTGCCACCTGCCTATACCAATAGCTTCACACGGGGCGTGTACTAT
CCCGACAAGGTGTTTAGATCTAGCGTGCTGCACTCCACCCAGGATCTGTTTCTGCCT
TTCTTTTCTAACGTGACATGGTTCCACGCCATCCACGTGTCCGGCACCAATGGCACA
AAGCGGTTCGACAATCCAGTGCTGCCCTTTAACGATGGCGTGTACTTCGCCTCCACC
GAGAAGTCTAACATCATCAGAGGCTGGATCTTTGGCACCACACTGGACAGCAAGAC
CCAGTCCCTGCTGATCGTGAACAATGCCACAAACGTGGTCATCAAGGTGTGCGAGT
TCCAGTTTTGTAATGATCCCTTCCTGGGCGTGTACTATCACAAGAACAATAAGTCTTG
GATGGAGAGCGAGTTTAGGGTGTATTCCTCTGCCAACAATTGCACCTTTGAGTACGT
GAGCCAGCCTTTCCTGATGGACCTGGAGGGCAAGCAGGGCAATTTCAAGAACCTGA
GGGAGTTCGTGTTTAAGAATATCGATGGCTACTTCAAGATCTACTCCAAGCACACAC
CAATCAACCTGGTGCGCGACCTGCCACAGGGCTTCTCTGCCCTGGAGCCACTGGTG
GATCTGCCCATCGGCATCAACATCACCCGGTTTCAGACACTGCTGGCCCTGCACAGA
AGCTACCTGACCCCAGGCGACAGCTCCTCTGGATGGACAGCAGGAGCTGCCGCCTA
CTATGTGGGCTATCTGCAGCCCCGCACCTTCCTGCTGAAGTACAACGAGAATGGCAC
CATCACAGACGCAGTGGATTGCGCCCTGGACCCCCTGTCTGAGACCAAGTGTACAC
TGAAGAGCTTTACAGTGGAGAAGGGCATCTACCAGACCAGCAACTTCAGGGTGCAG
CCAACAGAGTCCATCGTGCGCTTTCCCAATATCACCAACCTGTGCCCTTTTGGCGAG
GTGTTCAATGCCACACGCTTCGCCAGCGTGTACGCCTGGAATAGGAAGCGCATCTCC
AACTGCGTGGCCGACTATTCTGTGCTGTACAACAGCGCCTCCTTCTCTACCTTTAAGT
GTTATGGCGTGAGCCCCACCAAGCTGAATGATCTGTGCTTTACAAACGTGTACGCCG
ATTCCTTCGTGATCAGGGGCGACGAGGTGCGCCAGATCGCACCAGGACAGACCGGC
AAGATCGCAGACTACAATTATAAGCTGCCTGACGATTTCACAGGCTGCGTGATCGCC
TGGAACTCTAACAATCTGGATAGCAAAGTGGGCGGCAACTACAATTATCTGTACCGG
CTGTTTAGAAAGTCTAATCTGAAGCCATTCGAGCGGGACATCTCCACCGAGATCTAC

CAGGCCGGCTCTACACCCTGCAATGGCGTGGAGGGCTTTAACTGTTATTTCCCTCTG
CAGTCCTACGGCTTCCAGCCAACCAACGGCGTGGGCTATCAGCCCTACAGAGTGGT
GGTGCTGTCTTTTGAGCTGCTGCACGCACCTGCAACCGTGTGCGGCCCAAAGAAGA
GCACAAATCTGGTGAAGAACAAGTGCGTGAACTTCAACTTCAACGGACTGACCGGC
ACAGGCGTGCTGACCGAGAGCAACAAGAAGTTCCTGCCATTTCAGCAGTTCGGCAG
GGACATCGCAGATACCACAGACGCCGTGCGCGACCCTCAGACCCTGGAGATCCTGG
ACATCACACCATGTTCCTTCGGCGGCGTGTCTGTGATCACCCCAGGCACCAATACAT
CCAACCAGGTGGCCGTGCTGTATCAGGACGTGAATTGCACAGAGGTGCCCGTGGCA
ATCCACGCAGATCAGCTGACCCCTACATGGCGGGTGTACTCTACCGGCAGCAACGTG
TTCCAGACAAGAGCCGGATGCCTGATCGGAGCAGAGCACGTGAACAATAGCTATGA
GTGCGACATCCCTATCGGCGCCGGCATCTGTGCCTCCTACCAGACCCAGACAAACTC
CCCAAGGAGAGCCCGGTCTGTGGCCAGCCAGTCCATCATCGCCTATACCATGAGCCT
GGGCGCCGAGAACAGCGTGGCCTACTCCAACAATTCTATCGCCATCCCTACCAACTT
CACAATCAGCGTGACCACAGAGATCCTGCCAGTGAGCATGACCAAGACATCCGTGG
ACTGCACCATGTATATCTGTGGCGATTCCACAGAGTGTTCTAACCTGCTGCTGCAGTA
CGGCTCCTTTTGCACCCAGCTGAATAGAGCCCTGACAGGCATCGCCGTGGAGCAGG
ACAAGAACACCCAGGAGGTGTTCGCCCAGGTGAAGCAGATCTACAAGACACCACC
CATCAAGGACTTTGGCGGCTTCAACTTCAGCCAGATCCTGCCCGATCCTAGCAAGCC
ATCCAAGCGGTCTTTTATCGAGGACCTGCTGTTCAACAAGGTGACCCTGGCCGATGC
CGGCTTCATCAAGCAGTATGGCGATTGTCTGGGCGACATCGCCGCCAGAGACCTGAT
CTGCGCCCAGAAGTTTAATGGCCTGACCGTGCTGCCTCCACTGCTGACAGATGAGAT
GATCGCACAGTACACCTCTGCCCTGCTGGCCGGCACCATCACAAGCGGATGGACATT
CGGCGCAGGAGCCGCCCTGCAGATCCCCTTTGCCATGCAGATGGCCTATCGGTTCAA
CGGCATCGGCGTGACCCAGAATGTGCTGTACGAGAACCAGAAGCTGATCGCCAATC
AGTTTAACAGCGCCATCGGCAAGATCCAGGACTCTCTGAGCTCCACCGCCAGCGCC
CTGGGCAAGCTGCAGGATGTGGTGAATCAGAACGCCCAGGCCCTGAATACACTGGT
GAAGCAGCTGTCTAGCAACTTCGGCGCCATCTCCTCTGTGCTGAATGACATCCTGAG
CCGGCTGGACAAGGTGGAGGCAGAGGTGCAGATCGACCGGCTGATCACCGGCAGA
CTGCAGTCCCTGCAGACCTACGTGACACAGCAGCTGATCAGGGCAGCAGAGATCAG
GGCCTCTGCCAATCTGGCCGCCACAAAGATGAGCGAGTGCGTGCTGGGACAGTCCA
AGAGGGTGGACTTTTGCGGCAAGGGCTATCACCTGATGAGCTTCCCACAGTCCGCC
CCTCACGGAGTGGTGTTTCTGCACGTGACCTACGTGCCAGCCCAGGAGAAGAACTT
CACCACAGCCCCCGCCATCTGTCACGATGGCAAGGCCCACTTTCCTAGGGAGGGCG

TGTTCGTGAGCAACGGCACCCACTGGTTTGTGACACAGCGCAATTTCTACGAGCCA
CAGATCATCACCACAGACAATACCTTCGTGTCCGGCAACTGCGACGTGGTCATCGGC
ATCGTGAACAATACAGTGTATGATCCTCTGCAGCCAGAGCTGGACTCTTTTAAGGAG
GAGCTGGATAAGTACTTCAAGAATCACACCAGCCCCGACGTGGATCTGGGCGACAT
CTCTGGCATCAATGCCAGCGTGGTGAACATCCAGAAGGAGATCGACAGACTGAACG
AGGTGGCCAAGAATCTGAACGAGAGCCTGATCGATCTGCAGGAGCTGGGCAAGTAT
GAGCAGTACATCAAGTGGCCCTGGTATATCTGGCTGGGCTTCATCGCCGGCCTGATC
GCCATCGTGATGGTGACCATCATGCTGTGCTGTATGACAAGCTGCTGTTCCTGCCTG
AAGGGCTGCTGTTCTTGTGGCAGCTGCTGTAAGTTTGATGAGGACGATTCCGAGCCT
GTGCTGAAGGGCGTGAAGCTGCACTACACCTAA (SEQ ID NO.: 1).

In order to reduce the GC-content of the gene, the inventor further optimized the codons based on NB2, resulting in a sequence designated as NB4. NB4 has a sequence as shown by SEQ ID No.: 2:

ATGTTTGTCTTCCTGGTGCTGCTGCCTCTGGTCTCTTCTCAGTGTGTGAACCTGACCA
CCAGGACACAGCTGCCTCCTGCTTACACCAACTCCTTCACACGGGGCGTCTACTACC
CTGACAAGGTCTTCAGGTCCTCTGTGCTGCACTCCACACAAGACCTGTTCCTGCCAT
TCTTCTCCAACGTGACCTGGTTCCATGCCATCCATGTCTCTGGCACCAATGGCACCA
AGAGGTTCGACAACCCTGTGCTGCCATTCAACGACGGCGTCTACTTTGCCTCCACCG
AGAAGTCCAACATCATCAGGGGCTGGATCTTTGGCACCACCCTGGACTCCAAGACC
CAGTCCCTGCTGATTGTGAACAATGCCACCAACGTGGTGATCAAAGTCTGCGAGTTC
CAGTTCTGCAATGACCCATTCCTGGGCGTCTACTACCACAAGAACAACAAGTCCTGG
ATGGAGTCTGAGTTCAGGGTCTACTCCTCTGCCAACAACTGCACCTTTGAATATGTC
TCCCAGCCATTCCTGATGGACCTGGAGGGCAAGCAGGGCAACTTCAAGAACCTGAG
GGAGTTCGTCTTCAAGAACATCGACGGCTACTTCAAGATCTACTCCAAGCACACACC
CATTAATCTCGTCCGGGATCTGCCTCAGGGCTTCTCTGCCCTGGAGCCCCTGGTGGA
CCTGCCCATTGGCATCAACATCACACGCTTTCAGACCCTGCTGGCTCTGCACCGGTC
TTACCTGACACCTGGCGACTCCTCCTCTGGCTGGACAGCTGGCGCTGCTGCCTACTA
TGTCGGCTACCTGCAGCCTAGGACCTTCCTGCTGAAGTACAATGAGAATGGCACCAT
CACAGATGCTGTGGACTGTGCCCTGGACCCCCTGTCTGAGACCAAGTGCACCCTGA
AGTCCTTCACAGTCGAGAAGGGCATCTACCAGACCTCCAACTTCCGGGTGCAGCCC
ACAGAGTCCATTGTCAGGTTCCCCAACATCACCAACCTGTGCCCATTTGGCGAGGTC
TTCAATGCCACACGCTTTGCCTCTGTCTATGCCTGGAACAGGAAGAGGATCTCCAAC

TGTGTGGCTGACTACTCTGTGCTGTACAACTCTGCCTCCTTCTCCACATTTAAGTGCT
ATGGCGTCTCCCCCACCAAGCTGAATGACCTGTGCTTCACCAATGTCTATGCTGACT
CCTTTGTGATCCGGGGCGATGAAGTTAGGCAGATTGCCCCTGGCCAGACAGGCAAG
ATTGCTGACTACAACTACAAGCTGCCTGATGACTTCACCGGCTGTGTGATTGCCTGG
AACTCCAACAACCTGGACTCTAAAGTTGGCGGCAACTACAACTACCTGTACAGGCT
GTTCAGGAAGTCCAACCTGAAGCCATTTGAGAGGGACATCTCCACAGAGATCTACC
AGGCTGGCTCCACCCCATGCAATGGCGTGGAGGGCTTCAACTGCTACTTCCCCCTGC
AATCCTATGGCTTCCAACCCACCAATGGCGTGGGCTACCAGCCATACAGGGTGGTGG
TGCTGTCCTTTGAGCTGCTGCATGCCCCTGCCACCGTCTGTGGCCCCAAGAAGTCCA
CCAACCTCGTGAAGAACAAATGTGTGAACTTCAACTTCAACGGCCTGACAGGCACA
GGCGTGCTGACAGAGTCCAACAAGAAGTTCCTGCCATTCCAACAGTTTGGCAGGGA
CATTGCTGACACCACCGATGCTGTCAGGGACCCCCAGACCCTGGAGATCCTGGACAT
CACCCCATGCTCCTTTGGCGGCGTCTCTGTGATCACCCCTGGCACCAACACCTCTAA
TCAAGTCGCTGTGCTGTACCAGGACGTGAACTGCACAGAGGTTCCTGTGGCCATCC
ATGCTGACCAGCTGACCCCCACCTGGAGGGTCTACTCCACCGGCTCCAATGTCTTCC
AAACACGGGCTGGCTGCCTGATTGGCGCTGAGCATGTGAACAACTCCTATGAGTGT
GACATCCCCATTGGCGCTGGCATCTGTGCCTCCTACCAAACCCAGACCAACTCCCCT
CGGAGGGCTCGCTCTGTGGCTTCTCAGTCTATCATTGCCTACACCATGTCCCTGGGC
GCTGAGAACTCTGTGGCCTACTCCAACAACTCCATTGCCATCCCCACCAACTTCACC
ATCTCTGTGACCACCGAGATCCTGCCTGTCTCCATGACCAAGACCTCTGTGGACTGC
ACCATGTACATCTGTGGCGACTCCACAGAGTGCTCCAACCTGCTGCTGCAGTATGGC
TCCTTCTGCACCCAACTGAACAGGGCCCTGACAGGCATTGCTGTGGAGCAGGACAA
GAACACCCAGGAGGTCTTTGCCCAAGTCAAGCAGATCTACAAGACCCCCCCCATCA
AGGACTTTGGCGGCTTCAACTTCTCCCAAATCCTGCCTGACCCATCCAAGCCATCCA
AGAGGTCCTTCATTGAGGACCTGCTGTTCAACAAGGTTACCCTGGCTGATGCTGGCT
TCATCAAGCAGTATGGCGACTGCCTGGGCGACATTGCTGCCAGGGACCTGATCTGTG
CCCAAAAGTTCAATGGCCTGACAGTGCTGCCCCCCCTGCTGACCGATGAGATGATTG
CCCAGTACACATCTGCTCTGCTGGCTGGCACAATCACCTCTGGCTGGACCTTTGGCG
CTGGCGCTGCCCTGCAAATCCCATTTGCCATGCAAATGGCCTACAGGTTCAATGGCA
TTGGCGTGACCCAGAACGTGCTGTATGAGAACCAGAAGCTGATTGCCAACCAGTTC
AACTCTGCCATTGGCAAGATCCAGGACTCCCTGTCCTCCACCGCCTCTGCCCTGGGC
AAGCTGCAGGATGTGGTGAACCAGAATGCCCAGGCCCTGAACACCCTGGTGAAGCA
GCTGTCCTCCAACTTTGGCGCCATCTCCTCTGTGCTGAATGACATCCTGTCCCGGCTG

GACAAGGTGGAGGCTGAGGTGCAGATTGACAGGCTGATCACAGGCAGGCTGCAGT
CCCTGCAAACCTATGTGACCCAGCAGCTGATCAGGGCTGCTGAGATCAGGGCCTCT
GCCAACCTGGCTGCCACCAAGATGTCTGAATGTGTGCTGGGCCAGTCCAAGAGGGT
GGACTTCTGTGGCAAGGGCTACCATCTGATGTCCTTCCCCCAATCTGCCCCCCATGG
CGTGGTCTTCCTGCATGTGACCTATGTGCCTGCTCAGGAGAAGAACTTCACCACAGC
CCCTGCCATCTGCCATGATGGCAAGGCCCACTTCCCTCGGGAGGGCGTCTTTGTCTC
CAATGGCACCCACTGGTTTGTGACCCAGAGGAACTTCTATGAGCCTCAGATCATCAC
CACAGACAACACCTTTGTCTCTGGCAACTGTGATGTGGTGATTGGCATTGTGAACAA
CACAGTCTATGACCCCCTGCAGCCTGAGCTGGACTCCTTCAAAGAGGAGCTGGACA
AGTACTTCAAGAACCACACCTCCCCTGATGTGGACCTGGGCGACATCTCTGGCATCA
ATGCCTCTGTGGTGAACATCCAGAAGGAGATTGACAGGCTGAATGAGGTGGCCAAG
AACCTGAATGAGTCCCTGATTGACCTGCAAGAGCTGGGCAAGTATGAGCAGTACAT
CAAGTGGCCATGGTACATCTGGCTGGGCTTCATTGCTGGCCTGATTGCCATTGTGATG
GTGACCATCATGCTGTGCTGCATGACCTCCTGCTGCTCCTGCCTGAAAGGCTGCTGC
TCCTGTGGCTCCTGCTGCAAGTTTGATGAGGATGACTCTGAGCCTGTGCTGAAGGGC
GTGAAGCTGCACTACACCTAATAA (SEQ ID NO: 2)

Hereinafter, the technical solution of the present disclosure will be further illustrated in combination with the experiments.

### Construction of vectors, pVAX-NB1, pVAX-NB2 and pVAX-NB4, for the expression of the Spike gene:

Using NB1 and NB2 as templates, respectively, the NB1 fragment was obtained through PCR amplification with primers NB1-F and NB1-R, the NB2 fragment was obtained through PCR amplification with primers NB2-F and NB2-R, and the NB4 fragment was obtained through PCR amplification with primers NB4-F and NB4-R. The vector plasmid backbone pVAX was PCR amplified, by using CMV-R and BGH-F as primers, and using a pVAX plasmid, which was owned by our company, as the template. Then, two-fragment *in vitro* recombination was carried out by using a homologous recombination enzyme (Exnase) with the NB1, NB2 or NB4 fragment, respectively, to obtain pVAX-NB1, pVAX-NB2 and pVAX-NB4.

### Detection of Spike gene expression

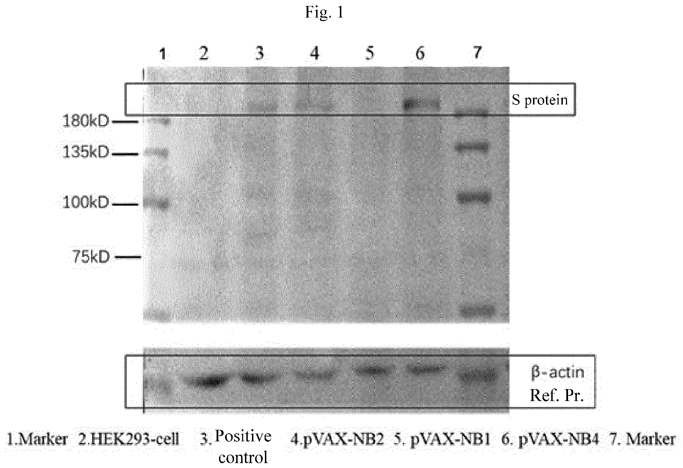

According to a conventional method, transfecting HEK293 cells with 2.5 µg of pVAX-NB1, pVAX-NB2 and pVAX-NB4, respectively, with cationic liposomes for 48 hours, and then collecting the cells. Such samples were processed according to a conventional Western Blot method, and were detected for the protein (FIG. 1). It could be seen that no S protein expression was detected in the pVAX-NB1 sample, while the expression of the S protein could be observed in the codon-optimized pVAX-NB2 and pVAX-NB4 samples. The expression level of S protein for pVAX-NB4 was about 2-3 times of that of S protein for pVAX-NB2. This indicates that the NB4 sequence produced unexpected effect.

### Animal immunogenicity experiments for DNA vaccines

Mice were intramuscularly injected with the S protein-expressing plasmids pVAX-NB1 and pVAX-NB2, 100 µg DNA/animal. In particular, 100 µg plasmids were added with 10% glucose to make it up to 200 µl in volume, and added with 10 µl Turbofect (Thermosfisher) according to the manufacturer's instructions. Orbital blood was collected 14 days after the immunization, and the antibody level in the serum was detected by adopting the S protein of the novel coronavirus as the antigen through enzyme-linked immunosorbent assay (ELISA). The specific process was as follows:
1) adding 100 µl of PBS and 50 ng of the S protein per well in a 96-well plate, incubating overnight at 4°C, for 16-18 h;
2) removing the supernatant, washing with PBST once, adding 200 µl of 5% skim milk for blocking at room temperature for 2 h;
3) washing with PBST twice;
4) adding the samples: adding 100 µl of diluted serum samples, incubating at 37°C for 2 h, and then washing with PBST for 5 times;
5) adding enzyme-labeled antibodies: adding 100 µl of diluted HRP-labeled IgM or IgG secondary antibody, and incubating at 37°C for 2 h;
6) washing with PBST for 6-8 times;
7) adding substrate solution for development: adding 100 µl of TMB for development;
8) terminating the reaction: adding with 50 µl of 1 M sulfuric acid to terminate the reaction; and
9) determining the results: determining the OD values, and controlling the OD values between 0.1 and 4.

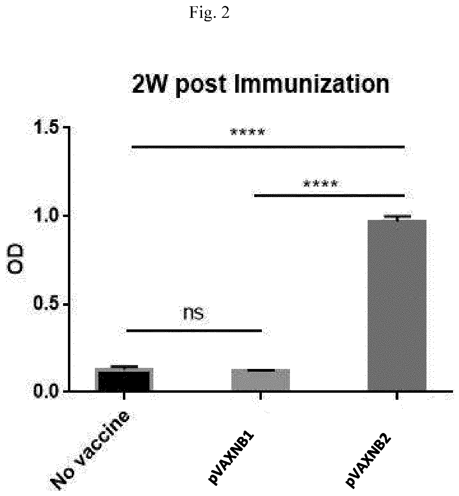

The experimental results showed that, NB2 could induce the generation of antibodies in the mice (FIG. 2).

## Claims

1. A nucleotide sequence encoding a peptide triggering immunogenicity against Novel Severe Acute Respiratory Syndrome Coronavirus 2 (SARS-CoV-2), comprising at least one selected from:
a) a nucleotide sequence as shown by SEQ ID NO: 1;
b) a nucleotide sequence as shown by SEQ ID NO: 2; or
c) a nucleotide sequence having at least 90% sequence identity with the nucleotide sequence as shown by SEQ ID NO: 1.

2. The nucleotide sequence according to claim 1, wherein the peptide is capable of:
inducing an immune response in a human body;
generating a molecular biology reporter;
generating a molecule for detection;
regulating a gene function; or
functioning as a therapeutic molecule.

3. An expression vector comprising the nucleotide sequence according to claim 1.

4. The expression vector according to claim 3, wherein the vector is a viral vector.

5. The expression vector according to claim 4, wherein the viral vector is an adenovirus vector.

6. A cell expressing a peptide based on the nucleotide sequence according to claim 1.

7. A nucleotide-based composition comprising the nucleotide sequence according to claim 1 or the expression vector according to claims 3-5.

8. The nucleotide-based composition according to claim 7, further comprising a pharmaceutically-acceptable adjuvant, carrier, diluent or excipient.

9. Use of the nucleotide-based composition according to claim 7 or 8 in the preparation of an agent for preventing or treating coronavirus disease 2019 (COVID-19).

10. A method for preventing or treating COVID-19, comprising administering a prophylactically or therapeutically effective amount of the nucleotide-based composition according to claim 7 or 8 to a human.
